Europäisches Patentamt

European Patent Office

Office européen des brevets

(19)

(11) Numéro de publication : **0 406 281 B1**

(12) **FASCICULE DE BREVET EUROPEEN**

(45) Date de publication du fascicule du brevet :
22.07.92 Bulletin 92/30

(51) Int. Cl.[5] : **C07K 15/06,** C07K 7/10,
G01N 33/68

(21) Numéro de dépôt : **89903756.8**

(22) Date de dépôt : **17.03.89**

(86) Numéro de dépôt international :
**PCT/FR89/00115**

(87) Numéro de publication internationale :
**WO 89/08668 21.09.89 Gazette 89/23**

(54) **PROTEINE DE RECONNAISSANCE DE LA GESTATION.**

(30) Priorité : **18.03.88 FR 8803590**

(43) Date de publication de la demande :
**09.01.91 Bulletin 91/02**

(45) Mention de la délivrance du brevet :
**22.07.92 Bulletin 92/30**

(84) Etats contractants désignés :
**AT BE CH DE FR GB IT LI LU NL SE**

(56) Documents cités :
**EP-A- 0 132 750**
**Biology of Reproduction, volume 26, 1982, J.E.**
**Butler et al.: "Detection and partial characteri-**
**zation of two bovinepregnancy-specific pro-**
**teins", pages 925-933.**

(56) Documents cités :
Chemical Abstracts, volume 102, 1985, (Columbus, Ohio, US), voir page 360, abrégé
109382b, & JP, A, 59214767 (GREEN CROS-
SCORP.) 4 décembre 1984
Chemical Abstracts, volume 87, 1977, (Columbus, Ohio, US), D.B. Laster: "A pregnancy-
specific protein in the bovine uterus"see page
254, abrégé 50654h, & Biol. Reprod. 1977,
16(5), 682-90

(73) Titulaire : **INSTITUT NATIONAL DE LA**
**RECHERCHE AGRONOMIQUE (INRA)**
**147, rue de l'Université**
**F-75341 Paris Cédéx 07 (FR)**

(72) Inventeur : **MARTAL, Jacques**
**5, allée du Josas**
**F-78350 Jouy-en-Josas (FR)**
Inventeur : **CAMOUS, Sylvaine**
**14, rue des Mortes-Fontaines**
**F-92370 Chaville (FR)**

(74) Mandataire : **Ores, Irène et al**
**CABINET ORES 6, Avenue de Messine**
**F-75008 Paris (FR)**

## Description

La présente invention est relative à une nouvelle protéine de reconnaissance de la gestation chez les Ruminants, et à son utilisation, notamment pour la détection précoce de la gestation, ainsi qu'à un procédé de préparation de ladite protéine.

L'implantation d'un embryon chez la vache n'a pas lieu avant le 20ème jour de gestation. Toutefois, pour survivre, le conceptus doit envoyer avant le 17ème jour, un message à l'organisme maternel pour empêcher la régression du corps jaune. Il existe donc, selon toute apparence, des substances embryonnaires produites ou stimulées par le conceptus, qui sont impliquées dans les mécanismes de reconnaissance de la gestation. BUTLER, HAMILTON, SASSER, RUDER, HASS et WILLIAMS (BIOL.REPROD. 26, 925, 1982) ont identifié et isolé une protéine B spécifique de la gestation. Cette protéine, isolée d'extraits de membranes placentaires bovines de foetus âgés de 25 à 270 jours, présente un poids moléculaire de l'ordre de 47 à 53 kDa et un point isoélectrique de 4,0 à 4,4. Cette protéine B a été obtenue à partir de membranes placentaires d'embryons ayant de 16 à 280 jours de gestation, prélevées chez des vaches gravides abattues, puis congelées -20°C. Les membranes ont été finement émincées à l'aide d'une lame de rasoir et ont été homogénéisées, puis la préparation a été centrifugée à 10000 g pendant 30 minutes à + 4°C. On a conservé le surnageant résultant.

L'extrait placentaire a été placé dans un bain de glace et la fraction qui a précipité entre 50 et 65 % de saturation par le sulfate d'ammonium, est dissoute dans du tampon Tris-HCl 0,01 M, pH 7,5 à 4°C et dialysée pendant 48 heures à 4°C contre ce tampon, avec une limite d'exclusion de 12 000-13 000 Da. Le dialysat est concentré, puis soumis à un processus de chromatographie par échange d'ions. Ce processus a été réalisé sur une colonne de DEAE-cellulose sur laquelle la protéine a été chargée dans du tampon Tris-HCl 0,01 M (pH 7,5) à un débit de 1 ml/min. et après élution des protéines non fixées, un gradient linéaire de NaCl de 0 à 0,3 M est appliqué. Les fractions collectées sont éluées. Celles qui présentent une activité antigénique spécifique de la gestation sont réunies, congelées et stockées à -20°C, une filtration sur gel est alors réalisée sur une colonne de Bio-Gel A-0,5 dont la limite d'exclusion est de 500 000 Daltons et la gamme de fractionnement comprise entre 10 000 et 500 000 Daltons. La protéine spécifique de la gestation récupérée par chromatographie par échange d'ions, est concentrée par ultrafiltration.

La Demande de Brevet européen 132 750 du 16 Juillet 1984, déposée en revendiquant une priorité américaine du 21 Juillet 1983, est limitée dans son objet à une méthode de détection de la gestation chez des mammifères, qui consiste à détecter la présence d'un immuncomplexe entre un anticorps anti-protéine B et un antigène, dans un fluide physiologique du mammifère gravide, extérieur au foetus, tandis que la protéine B est marquée, de préférence par conjugaison, de manière à fournir un signal détectable.

Les Inventeurs se sont donné pour but de pourvoir à une protéine de reconnaissance de la gestation chez les Ruminants qui présente un haut degré de pureté, parfaitement identifiée et dont la préparation est rigoureusement reproductible ; les Inventeurs se sont également donné pour but de mettre en place des moyens, et en particulier ladite protéine, pour la réalisation d'un diagnostic précoce et spécifique de la gestation et d'un diagnostic de mortalité embryonnaire ; les Inventeurs se sont également donné pour but,en préparant une telle protéine pure et parfaitement identifiée,d'élucider les propriétés physiologiques de cette molécule.

La présente invention a pour objet une proteine spécifique de la gestation, analogue à la PSPB - Pregnancy Specific protéin B de Sasser - qui sera dénommée dans ce qui va suivre PSP$_{60}$, (ou Pregnancy Serum Protéine, de poids moléculaire d'environ 60 kDa), qui est caractérisée en ce qu'elle présente la séquence N-terminale en amino-acides représentée par la formule I ci-après :

$$-X-Gly-Ser-X-Leu-Thr-Thr-His-Pro-Leu-Arg-Asn-Ile-Lys-Asp-$$
$$\quad 1 \qquad\qquad 5 \qquad\qquad\qquad\qquad 10 \qquad\qquad\qquad\qquad\qquad 15$$

$$-Leu-Val-Tyr-Met-Gly-X-Ile-Thr-Ile-Gly-Thr-Pro-Pro-Gln-$$
$$\qquad\qquad\qquad 20 \qquad\qquad\qquad\qquad 28$$

$$-Glu-Phe-Gln-Val-Val-Phe-Asp-Thr-Ala-Ser-X- \qquad\qquad (I)$$
$$\quad 30 \qquad\qquad\qquad 35 \qquad\qquad\qquad 40$$

(les X étant probablement des asparagines).

Conformément à la présente invention, la PSP$_{60}$ présente un poids moléculaire de 60 kDa environ (par analyse par électrophorèse sur gel de polyacrylamide en milieu dénaturant) et un point isoélectrique de 5,1 à 5,5 environ,après électrophorèse bidimensionnelle.

La présente invention a, de plus, pour objet un procédé de préparation de la PSP$_{60}$ à partir de placentas de mammifères, qui est caractérisé en ce que des placentas homogénéisés et extraits dans un tampon appro-

2

prié, sont soumis à une précipitation acide et le surnageant est soumis à une précipitation saline, puis le précipité est soumis à une chromatographie sur échangeur d'ions puis à une filtration sur gel, les fractions présentant une activité $PSP_{60}$ sont purifiées par passage sur une colonne d'HPLC échangeuse de cations, suivi d'une élution par un gradient salin dans un tampon approprié à pH acide, ce qui permet d'isoler la $PSP_{60}$.

Conformément à la présente invention, la $PSP_{60}$ peut être obtenue par d'autres voies, notamment par d'autres voies de purification des protéines ou par voie biotechnologique (y compris par génie génétique, synthèse bactérienne ou cellulaire, animaux transgéniques, etc..)

La présente invention a en outre pour objet un kit de réalisation d'un test de diagnostic précoce de gestation, chez les mammifères et principalement le ruminant, qui est caractérisé en ce que, outre les réactifs, tampons et/ou diluants usuels, il comprend une quantité utile d'anticorps polyclonaux ou monoclonaux anti-$PSP_{60}$.

Conformément à l'invention, les anticorps sont obtenus soit à partir de sérums de mammifères immunisés par la $PSP_{60}$ soit par fusion de cellules de mammifères immunisés par la $PSP_{60}$ avec des cellules malignes de mammifères appropriées (tels que la souris, notamment).

Outre les dispositions qui précèdent, l'invention comprend encore d'autres dispositions, qui ressortiront de la description qui va suivre.

L'invention sera mieux comprise à l'aide de complément de description qui va suivre qui se réfère à des exemples de mise en oeuvre de l'objet de la présente invention.

Il doit être bien entendu, toutefois, que ces exemples de mise en oeuvre sont donnés uniquement à titre d'illustration de l'objet de l'invention, dont ils ne constituent en aucune manière une limitation.

La PSPB a été mise en évidence par une équipe américaine (BUTLER et al., 1982), mais de nombreux problèmes n'étaient pas résolus : fiabilité et limites de l'application du dosage de la PSPB au diagnostic de gestation, intérêt dans l'estimation de la mortalité embryonnaire, source réelle de sécrétion, caractéristiques biochimiques précises de la molécule dont les préparations les plus pures (préparations de radioiodation) étaient hétérogènes, des immunsérums dirigés contre des homogénats de trophoblaste ou des préparations protéiques insuffisamment purifiées, méthode de purification imprécise et/ou dénaturante (isoélectrofocalisation) présence mal connue dans la circulation du foetus et dans les liquides des enveloppes embryonnaires, propriétés biologiques totalement ignorées. Par ailleurs, on disposait d'un dosage radioimmunologique (cf. demande de brevet européen précitée) utilisable pour le dosage de protéines mal définies, spécifique de la gestation chez les bovins. Le dosage de la PSPB présentait, cependant, un grand intérêt puisqu'il devait permettre d'effectuer des diagnostics spécifiques de gestation. En effet, le dosage de la progestérone, reflet de l'activité lutéale, largement répandu actuellement, constitue un excellent moyen de diagnostic de non gestation, mais il peut être à l'origine d'un certain nombre de faux diagnostics positifs de gestation dans le cas de corps jaunes persistants à la suite de mortalité embryonnaire (à partir du 16ème jour chez la vache). De plus, en raison de la sécrétion de la progestérone durant chaque phase lutéale du cycle oestrien, il est indispensable de connaître rigoureusement la date de la saillie ou de l'insémination artificielle, ce qui n'est pas toujours le cas (troupeaux bovins de races à viande, par exemple).

Exemple 1

Purification de la $PSP_{60}$ bovine par HPLC.

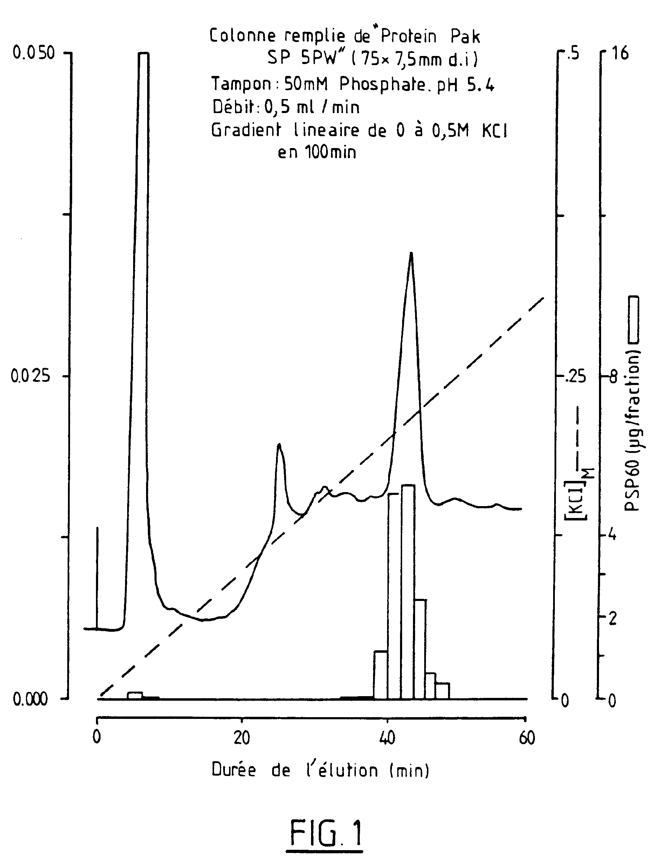

Des cotylédons foetaux congelés ont été homogénéisés et extraits dans du tampon phosphate KCl (0,1 M) pH 8,6. L'extrait placentaire a été soumis à une précipitation acide (à pH 4,5) et le surnageant a été précipité par du sulfate d'ammonium (40 % et 65 % de saturation pH 5,2).Le précipité a ensuite été dialysé et soumis à une chromatographie sur DEAE-Sephadex, puis à une filtration sur gel. La présence de $PSP_{60}$ dans les fractions a été déterminée par radioimmunologie à l'aide de préparations de PSPB impures telles que décrites dans la Publication BUTLER et al. Les fractions PSP- positives ont été appliquées sur une colonne d'HPLC échangeuse de cations (TSK SP 5 PW ; 75 mm x 7,5 mm diamètre intérieur) et des protéines ont été éluées par un gradient linéaire de KCl dans du tampon phosphate 0,05 M à pH 5,4 en colonne analytique ou à pH 5,85 en colonne préparative. La figure 1 annexée montre un pic majeur distinct, ($PSP_{60}$) élué de la colonne d'HPLC à la concentration de 0,195M de KCl, présente une réactivité immunologique dans le test de dosage radioimmunologique de la PSPB proposé par SASSER et al.. Par analyse par électrophorèse en gel de polyacrylamide en milieu dénaturant, ce pic de $PSP_{60}$ correspond à une seule bande et à un poids moléculaire apparent de 60 kDa environ. Après radioiodation, cette protéine précipite avec l'immunsérum anti-PSPB et présente la même mobilité électrophorétique que l'une des protéines contenue dans la préparation de PSPB radioiodée, obtenue par BUTLER, SASSER et al. Cependant, les caractéristiques de la PSPB de BUTLER et al., $\simeq$ 47 - 53 kDa, point isoélectrique 4-4,4, font apparaître qu'il ne s'agit pas dans la présente invention de la même molécule, puisque la $PSP_{60}$ a un P.M. de 60 kDa et un point isoélectrique de 5,1-5,5.

Exemple 2 Caractérisation de la $PSP_{60}$ bovine.

## 2.1 Caractérisation physicochimique.

Après analyse par électrophorèse sur gel d'acrylamide en milieu dénaturant, le pic de $PSP_{60}$ purifiée par HPLC ne présente qu'une seule bande de PM apparent de 60 kDa (au lieu de 47 à 53 kDa par BUTLER et al., 1982). Cependant, dans les mêmes conditions expérimentales, la PSPB de SASSER présente, après radioiodation, une protéine de PM apparent de 59 kDa et une autre mineure de 51 kDa. Le point isoélectrique de la $PSP_{60}$ purifiée par HPLC a été déterminé à 5,5 après isoélectrofocalisation. Il diffère donc sensiblement de celui de la PSPB décrite par SASSER et BUTLER. La protéine $PSP_{60}$ purifiée conformément à l'invention est néanmoins précipitable par l'immunsérum anti-PSPB de SASSER.

## 2.2 Caractérisation par système de traduction acellulaire

La capacité synthétique d'ARNm poly(A)$^+$ d'embryons ovins âgés de 29 jours dans un système acellulaire (lysat de réticulocytes) en présence de $^{35}S$-méthionine a été déterminée. La quantité de $PSP_{60}$ synthétisée a été estimée par immunoprécipitation spécifique à l'aide d'immunsérum anti-PSPB bovine, suivie d'électrophorèse-SDS : le précurseur de la $PSP_{60}$ correspond à une protéine de 49 kDa, dont le FM apparent est nettement inférieur à celui de la $PSP_{60}$ (60 kDa), ce qui signe très probablement la nature glycoprotéique de la proteine native. Cette traduction de la $PSP_{60}$ en milieu acellulaire suggère que sa fraction protéique sécrétée présente un PM apparent d'environ 46 kDa.

## Exemple 3

## Dosage radioimmunologique de la $PSP_{60}$.

Une méthode quantitative de détection de la $PSP_{60}$ a été mise au point par les Inventeurs. Elle consiste en un dosage radioimmunologique, utilisant deux anticorps, dans les conditions suivantes : la $PSP_{60}$ utilisée pour le marquage à l'Iode 125 et pour les courbes d'étalonnage, est issue de la dernière étape de purification par chromatographie HPLC. Un antisérum anti-$PSP_{60}$, obtenu après immunisation de lapins, est utilisé à la dilution finale de 1/1 600 000 ; il lie 25 à 30 % de la $PSP_{60}$ radioiodée dans les conditions de dosage établies par les Inventeurs.

## Caractéristiques du dosage :

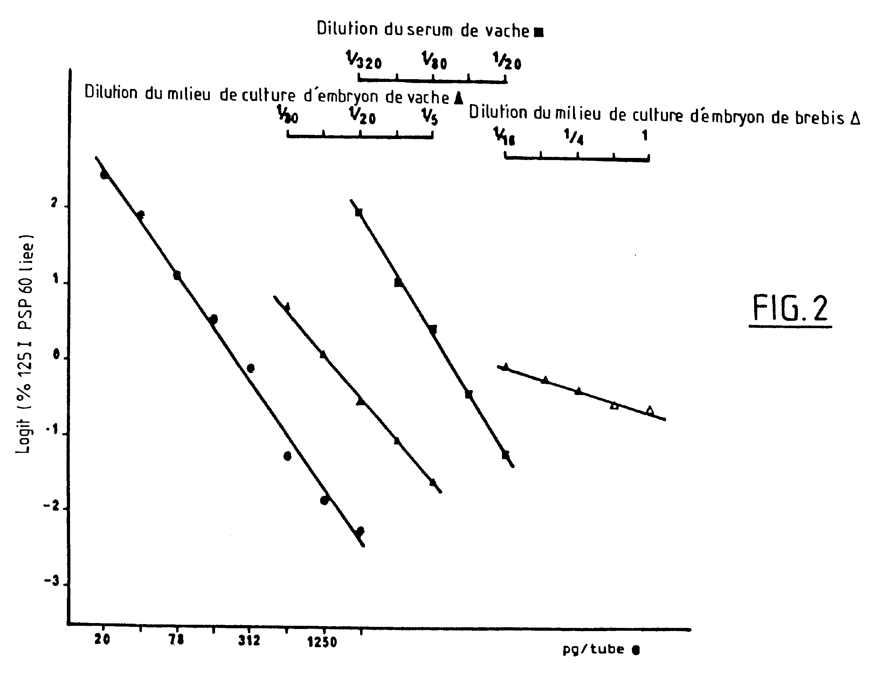

– sensibilité : cette méthode permet de détecter 20 pg de $PSP_{60}$ dans 100 µl d'échantillon ;
– la reproductibilité intra- et inter-dosage, exprimée par le coefficient de variation, est de 6 % et 12 % respectivement pour un échantillon de sérum contenant 1,3 ng/ml de $PSP_{60}$ ;
– parallélisme : le sérum ou le plasma de vache gestante et le milieu de culture d'embryons bovins présentent des courbes doses-réponses parallèles à la courbe de référence, comme le montre la figure 2 qui représente la courbe d'inhibition de la liaison $PSP_{60}$ radioiodée/anticorps anti-$PSP_{60}$ obtenue par des dilutions croissantes de sérum ou de milieu de culture. Les autres composés présents dans le sang, ou sécrétés dans le milieu de culture par les embryons, n'interfèrent donc pas avec le dosage de la $PSP_{60}$.
Le sérum de brebis gestante et le milieu de culture d'embryon ovin croisent avec la $PSP_{60}$, mais de façon non parallèle (voir figure 2).
– spécificité du dosage : il n'y a pas de réaction croisée avec l'$\alpha$ foeto protéine bovine.

## Exemple 4

## Caractérisation de la sécrétion de $PSP_{60}$

## Sécrétion in vitro de la $PSP_{60}$ par l'embryon

Grâce au dosage décrit à l'Exemple 3, on peut suivre l'évolution de la sécrétion de $PSP_{60}$ par des embryons cultivés in vitro. Chez la vache, la $PSP_{60}$ est présente dès le 16ème jour de gestation dans les milieux de culture, alors qu'on ne la détecte pas encore dans le sang. A 25 Jours de gestation, une dizaine d'explants de tissus trophoblastiques (environ 0,3 mg de protéine) sécrète plus d'une centaine de nanogrammes par 25 heures. Chez la brebis, la $PSP_{60}$ n'est pas décelée dans des milieux de culture d'embryons de 14 jours, mais dès 15

**4**

jours de gestation elle est détectable (24 à 180 ng/embryon entre 15 et 24 jours de gestation) avec un dosage hétérologue. Le dosage de la $PSP_{60}$ bovine ne permet donc pas chez la brebis de déterminer de façon précise les quantités sécrétées par l'embryon.

Localisation cellulaire de la $PSP_{60}$ par immunocytofluorescence.

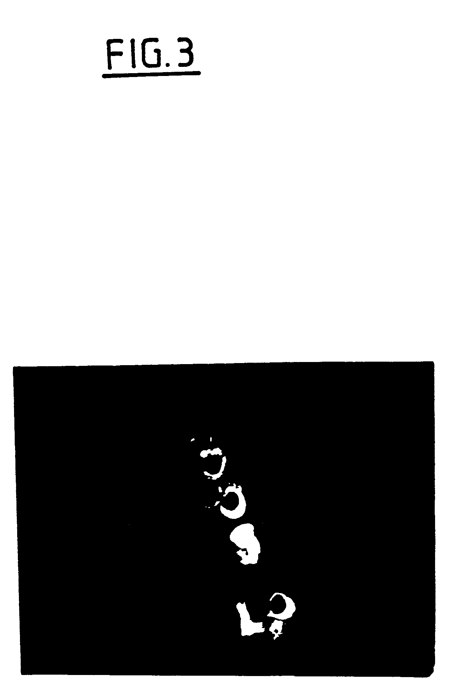

La technique d'immunocytofluorescence met en évidence une localisation spécifique de la $PSP_{60}$ dans le cytoplasme des cellules géantes binucléées du trophoblaste de brebis à 24 jours de gestation (les autres types cellulaires ne sont pas marqués). La figure 3 reproduit une photographie de l'allantochorion de brebis au $24^{ème}$ jour ; la localisation de la $PSP_{60}$ est réalisée par immunocytofluorescence dans les cellules binucléées.

Sécrétion au cours de la gestation chez la vache

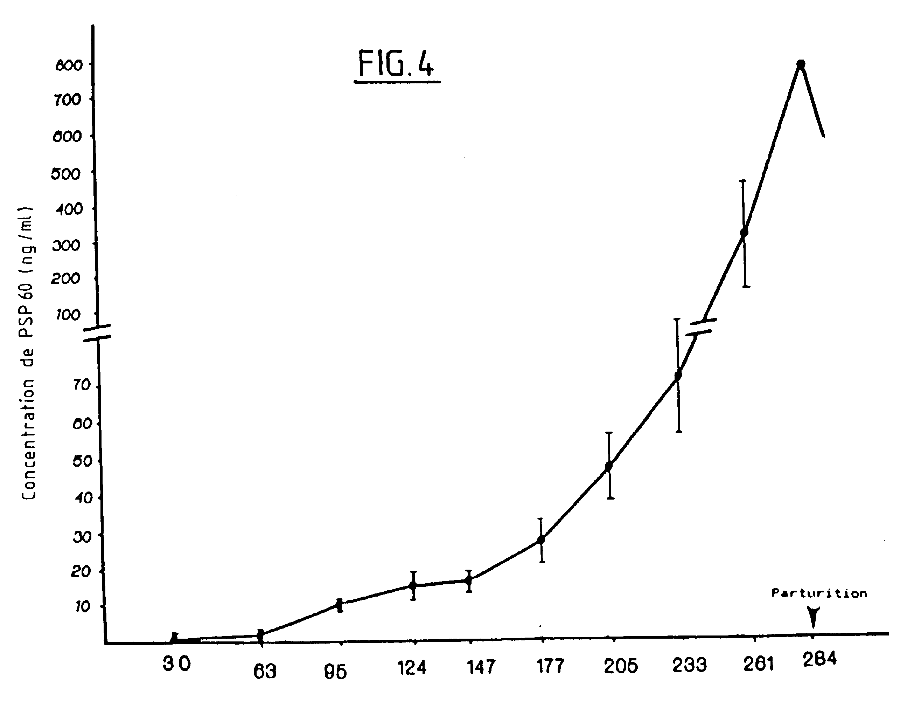

La $PSP_{60}$ apparaît dans le sang maternel vers le $25^{ème}$-$30^{ème}$ jour de gestation. Elle augmente progressivement jusqu'à une trentaine de jours avant la parturition : 1,9 ± 0,58 ng/ml (x ± SE) à 63 jours (n = 3) ; 10,6 ± 1,67 ng/ml à 95 jours (n = 4) et 71,1 ± 16,1 ng/ml à 233 jours (n = 8). Un mois environ avant la parturition, le niveau de $PSP_{60}$ s'élève brutalement (314 ± 151 ng/ml à 261 jours, n = 8) pour atteindre son maximum à la parturition (790 ng/ml chez une vache), (figure 4).

Vitesse de disparition de la $PSP_{60}$ post partum

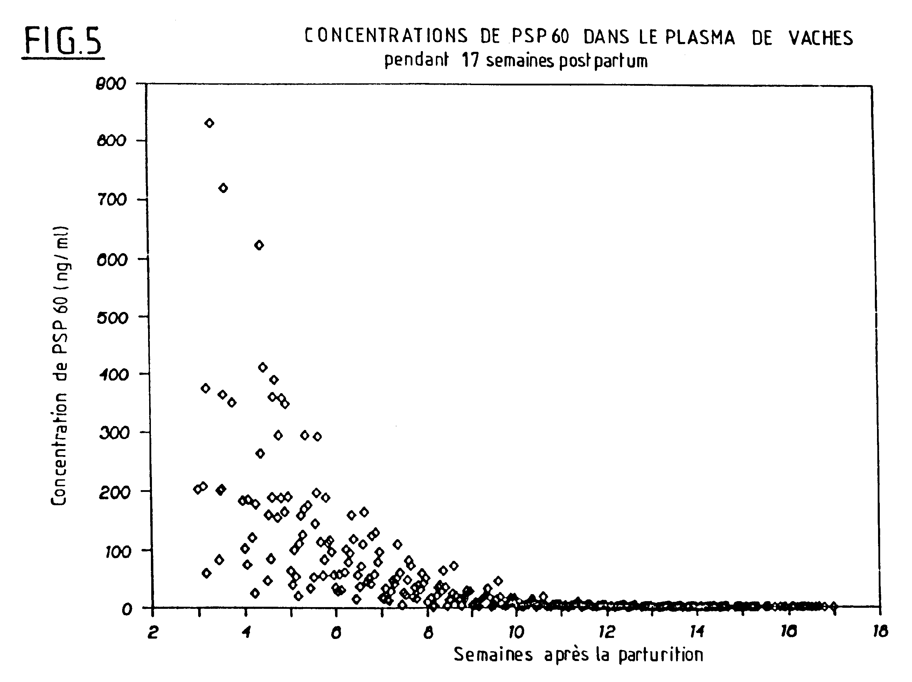

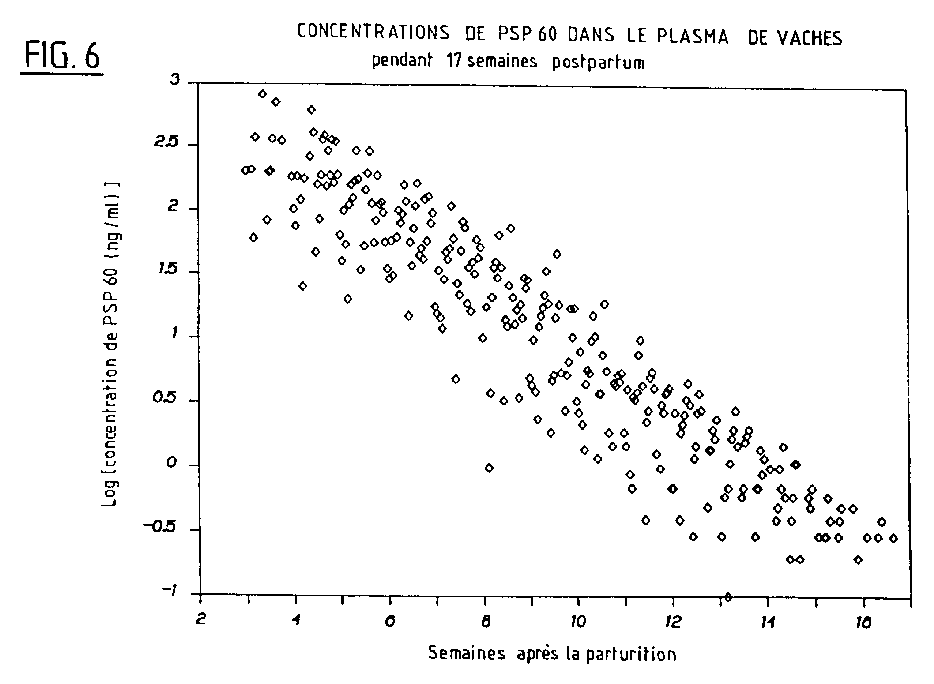

La $PSP_{60}$ est présente dans la circulation maternelle à des taux non négligeables jusqu'à approximativement 10 semaines après la parturition, ensuite les taux sont proches de zéro (figure 5). Le dosage de la $PSP_{60}$, chaque semaine, entre 3 et 16 semaines post partum chez 25 vaches, a permis d'établir la droite de régression du logarithme de la concentration de $PSP_{60}$ en fonction du temps (en semaines) (figure 6).

$$Y = -0,25 x + 3,24$$

Ainsi la demi-vie de la $PSP_{60}$ a été estimée à 8,4 jours.

Exemple 5

Diagnostic de gestation par le dosage de la $PSP_{60}$

Sur 61 vaches et 79 génisses, un premier diagnostic de gestation par dosage de la $PSP_{60}$ a été réalisé à 25,26 ou 27 jours après insémination artificielle. Afin de confirmer le diagnostic, une palpation rectale et un prélèvement de sang pour le dosage de la $PSP_{60}$ ont été effectués à 90 jours sur tous les animaux revenus en chaleurs. L'exactitude des diagnostics positifs (nombre de diagnostics positifs exacts/nombre total de dioagnostics positifs) est voisine de 78 % à 25 et 26 jours et dépasse 90 % à 27 jours chez les vaches, alors qu'à ces 3 stades l'exactitude des diagnostics positifs, chez les génisses est supérieure à 90 %, ainsi que cela ressort du tableau I qui va suivre.

EP 0 406 281 B1

## TABLEAU I.

### PRECISION DU DIAGNOSTIC PAR RIA AVEC LA $PSP_{60}$
### 25, 26 OU 27 JOURS APRES I A

### PRECISION DES RESULTATS POSITIFS

| | Jour 25 | | Jour 26 | | Jour 27 | | Jours 25 à 27 | | |
|---|---|---|---|---|---|---|---|---|---|
| | % | n | % | n | % | n | % | n | |
| VACHES | 78,3 | (18/23) | 78,9 | (15/19) | 94,7 | (18/19) | 83,6 | (51/61) | b |
| GENISSES | 91,7 | (22/24) | 95,6 | (22/23) | 96,9 | (31/32) | 94,9 | (75/76) | c |
| TOTAL | 85,1 | (40/47) | 88,1 | (37/42) | 96,1 | (49/51) | | | |

b/c $p < 0,05$

Précision des résultats positifs = A/B

A = nombre d'animaux testés positifs par le test à la $PSP_{60}$ et par palpation rectale au jour 90.

B = nombre d'animaux testés positifs par le test à la $PSP_{60}$

Dans tous les cas, l'exactitude augmente avec le stade de prélèvement et est significativement plus élevée chez les génisses que chez les vaches (P<0,05). Seulement quatre vaches ont été inséminées avant le 70ème jour après vélage et présentent une concentration résiduelle de $PSP_{60}$ post partum qui implique un diagnostic de gestation erroné.

Exemple 6

Mise en évidence de mortalités embryonnaires par le dosage de la $PSP_{60}$

Chez les vaches ou les génisses qui présentent une mortalité embryonnaire tardive (cf tableau II ci-après), le dosage de la $PSP_{60}$ à 26, 35, 50 et 90 jours après insémination artificielle met en évidence une décroissance des concentrations de $PSP_{60}$ et permet de situer le moment de cette mortalité embryonnaire, confirmée par échographie et palpation rectale.

EP 0 406 281 B1

## TABLEAU II.

### MORTALITE EMBRYONNAIRE

| VACHE | PSP$_{60}$ au jour 26 (ng/ml) | PSP$_{60}$ au jour 35 (ng/ml) | PSP$_{60}$ au jour 50 (ng/ml) | ECHOGRAPHIE au jour 50 | PSP$_{60}$ au jour 90 (ng/ml) | PALPATION RECTALE au jour 90 |
|---|---|---|---|---|---|---|
| 5013 | 0,7 | 2,2 | 2,8 | + | 1,1 | − |
| 5017 | 0 | 1,4 | 0,6 | − | 0 | − |
| 5041 | 0,3 | 4,7 | 2,8 | + | 0 | − |
| 5058 | 0 | 0,3 | 0 | − | | |
| 5067 | 0,8 | 1,2 | 0,4 | − | | − |
| 5094 | 0 | 0,8 | 0,3 | − | 0 | − |
| **GENISSE** | | | | | | |
| 6234 | 0 | 1,3 | 0,9 | + viabilité ? | | − |
| 6298 | 0,5 | 0 | vue dans l'oestrus | | | |

La PSP$_{60}$ identifiée et isolée conformément à la présente invention comporte de nombreuses applications, telles que : utilisation à des fins de diagnostic de gestation, de diagnostic de viabilité ou de mortalité embryonnaire, de détermination de la fertilité à des fins de sélection génétique.

De plus, l'approfondissement des connaissances relatives à la PSP$_{60}$ identifiée conformément à la présente invention permettra de préciser ses propriétés physiologiques et leurs éventuelles applications.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente invention.

**Revendications**

1. Proteine spécifique de la gestation chez les mammifères, dénommée PSP$_{60}$ caractérisée en ce qu'elle présente la séquence N-terminale en amino-acides représentée par la formule I ci-après :

```
-X-Gly-Ser-X-Leu-Thr-Thr-His-Pro-Leu-Arg-Asn-Ile-Lys-Asp
 1            5          (I)        10                  15
-Leu-Val-Tyr-Met-Gly-X-Ile-Thr-Ile-Gly-Thr-Pro-Pro-Gln-
                  20                      28
-Glu-Phe-Gln-Val-Val-Phe-Asp-Thr-Ala-Ser-X-        (I)
  30                35                  40
```

2. Proteine spécifique de la gestation chez les mammifères, dénommée PSP$_{60}$ caractérisée en ce qu'elle présente un poids moléculaire de 60 kDa environ (par analyse électrophorétique sur gel de polyacrylamide en milieu dénaturant), et un point isoélectrique d'environ 5,5 après électrophorèse bidimensionnelle.

3. Procédé de préparation de la PSP$_{60}$ à partir de placentas de mammifères, caractérisé en ce que des placentas homogénéisés et extraits dans un tampon approprié, sont soumis à une précipitation acide et le surnageant est soumis à une précipitation saline, puis le précipité est soumis à une chromatographie sur échangeur d'ions, puis à une filtration sur gel, les fractions présentant une activité PSP$_{60}$ sont purifiées par passage sur une colonne d'HPLC échangeuse de cations, suivi d'une élution par un gradient salin dans un tampon approprié à pH acide, ce qui permet d'isoler la PSP$_{60}$.

4. Kit de réalisation d'un test de diagnostic précoce de gestation ou de diagnostic de mortalité embryonnaire, caractérisé en ce que, outre les réactifs, tampons et/ou diluants usuels, il comprend une quantité utile d'anticorps polyclonaux ou monoclonaux anti-PSP$_{60}$.

**Claims**

1. Mammalian pregnancy-specific protein, referred to as PSP$_{60}$, characterised in that it has the N-terminal amino acid sequence represented by formula I below:

```
-X-Gly-Ser-X-Leu-Thr-Thr-His-Pro-Leu-Arg-Asn-Ile-Lys-Asp-
 1          5                    10                      15
Leu-Val-Tyr-Met-Gly-X-Ile-Thr-Ile-Gly-Thr-Pro-Pro-Gln-Glu-
                  20                28                  30
Phe-Gln-Val-Val-Phe-Asp-Thr-Ala-Ser-X-              (I)
              35                  40
```

2. Mammalian pregnancy-specific protein, referred to as PSP$_{60}$, characterised in that it has a molecular

9

weight of about 60 kDa (when analysed by polyacrylamide gel electrophoresis in a denaturing medium) and an isoelectric point of about 5.5 after two-dimensional electrophoresis.

3. Method of preparing $PSP_{60}$ from mammal placentas, characterised in that placentas, homogenised and extracted in an appropriate buffer, are subjected to acid precipitation and the supernatant is subjected to saline precipitation, the precipitate is then subjected to chromatography on an ion exchanger and subsequently to gel filtration, and the fractions possessing a $PSP_{60}$ activity are purified by passage over a cation exchange HPLC column, this being followed by elution with a saline gradient in an appropriate buffer at acid pH, which enables the $PSP_{60}$ to be isolated.

4. Kit for carrying out a test for the early diagnosis of pregnancy or the diagnosis of embryonic mortality, characterised in that, in addition to the customary reagents, buffers and/or diluents, it comprises a useful amount of anti-$PSP_{60}$ polyclonal or monoclonal antibodies.

## Patentansprüche

1. Für die Trächtigkeit bei Säugetieren spezifisches Protein mit der Bezeichnung $PSP_{60}$, dadurch gekennzeichnet, daß es die N-terminale Aminosäuresequenz der folgenden Formel I umfaßt:

$$-X-Gly-Ser-X-Leu-Thr-Thr-His-Pro-Leu-Arg-Asn-Ile-Lys-Asp$$
$$1 \qquad 5 \qquad \textbf{(I)} \qquad 10 \qquad 15$$
$$-Leu-Val-Tyr-Met-Gly-X-Ile-Thr-Ile-Gly-Thr-Pro-Pro-Gln-$$
$$20 \qquad 28$$
$$-Glu-Phe-Gln-Val-Val-Phe-Asp-Thr-Ala-Ser-X- \qquad \textbf{(I)}$$
$$30 \qquad 35 \qquad 40$$

2. Für die Trächtigkeit bei Säugetieren spezifisches Protein mit der Bezeichnung $PSP_{60}$, dadurch gekennzeichnet, daß es ein Molekulargewicht von etwa 60 kDA (mittels elektrophoretischer Analyse auf Polyacrylamidgel in denaturierendem Medium) und einen isoelektrischen Punkt von etwa 5,5 nach zweidimensionaler Elektrophorese zeigt.

3. Verfahren zur Herstellung von $PSP_{60}$, ausgehend von Placenten von Säugetieren, dadurch gekennzeichnet, daß die homogenisierten und in einem geeigneten Puffer extrahierten Placenten einer sauren Präzipitation unterworfen werden und der Überstand einer Salzpräzipitation unterworfen wird, anschließend das Präzipitat einer Ionenaustauschchromatographie, anschließend einer Gelfiltration unterworfen wird, die Fraktionen mit einer $PSP_{60}$-Aktivität durch Passage über eine Kationenaustauscher-HPLC-Säule mit anschließender Elution in einem Salzgradienten in einem geeigneten Puffer bei einem sauren pH, welcher die Isolierung von $PSP_{60}$ ermöglicht, gereinigt werden.

4. Kit zur Durchführung eines Tests zur vorzeitigen Diagnose der Trächtigkeit oder zur Diagnose der embryonalen Sterblichkeit, dadurch gekennzeichnet, daß er außer den üblichen Reagentien, Puffern und/oder Verdünnungsmitteln eine geeignete Menge eines polyklonalen oder monoklonalen Anti-$PSP_{60}$-Antikörpers enthält.

Colonne remplie de "Protein Pak
SP 5PW" (75× 7,5mm d.i)
Tampon: 50mM Phosphate. pH 5.4
Débit: 0,5 ml / min
Gradient linéaire de 0 à 0,5M KCl
en 100min

Durée de l'élution (min)

## FIG.1

FIG.2

EP 0 406 281 B1

# FIG.3

FIG.4

EP 0 406 281 B1

FIG.5

CONCENTRATIONS DE PSP 60 DANS LE PLASMA DE VACHES
pendant 17 semaines postpartum

Concentration de PSP 60 (ng/ml)

Semaines après la parturition

EP 0 406 281 B1

FIG. 6

CONCENTRATIONS DE PSP 60 DANS LE PLASMA DE VACHES
pendant 17 semaines postpartum

Log [concentration de PSP 60 (ng/ml)]

Semaines après la parturition

EP 0 406 281 B1